# EUROPEAN PATENT SPECIFICATION

(11) **EP 1 013 636 B1**
(45) Date of publication and mention of the grant of the patent: **09.07.2003**
(21) Application number: 99310181.5
(22) Date of filing: 17.12.1999
(51) Int. Cl.: C07C 205/06, C07C 205/43, C07C 255/60, C07C 205/12, C07C 205/45, C07C 25/24, C07C 205/37, C07C 205/56, C07D 213/61, A61K 49/04, C07C 201/12, C07C 253/30, C07C 17/26

(54) **Preparation of allylic aromatic compounds**
Herstellung von allylischen aromatischen Verbindungen
Préparation de composés allyliques aromatiques

(30) Priority: 18.12.1998 GB 9828103
(43) Date of publication of application: 28.06.2000
(73) Proprietor: Amersham Health AS, 0401 Oslo (NO)
(72) Inventor: Ek, Fredrik, 224 64 Lund (SE); Wistrand, Lars Göran, Malmö (SE)
(74) Representative: Canning, Lewis R.

(56) References cited:
- WO-A-96/09282
- US-A- 5 264 610
- JACEK PORWISIAK, MANFRED SCHLOSSER: "1-Bromo-3,5-bis(trifluoromethyl)benzene: A versatile starting material for organometallic synthesis." CHEMISCHE BERICHTE., vol. 129, 1996, pages 233-235, XP002133846 VERLAG CHEMIE GMBH. WEINHEIM., DE ISSN: 0009-2940

## Description

This invention is concerned with the preparation of allylic aromatic compounds.

A number of methods are known for the introduction of an allylic group into an aromatic compound, such as cross-coupling or direct allylation, but these often require the use of expensive and toxic metal reagents or catalysts, e.g. Sn or Pd, or air (oxygen) and water sensitive reagents and substrates which are difficult to work with on a large scale. It has often been shown that aromatic compounds substituted with electron withdrawing groups produce allylated products after extended reaction times and in reduced yield. Allylation of an aromatic carbon via a radical intermediate (e.g. a phenyl radical) has also been proposed (e.g. Migita et al., Bull. Chem. Soc. Jpn, 56, 2480-2484 (1983)), but these reactions require either at least two process steps and unstable and toxic intermediates or the use of toxic tin catalysts.

These methods are therefore unsuitable for use on a large scale, particularly in the manufacture of compounds required as intermediates for use in the production of medical products. An important example of an allylic compound of this kind is allyl-3,5-dinitrobenzene, which can be used in the production of iodinated X-ray contrast agents containing dihydroxypropyl side chains, such as described in WO 96/09282.

We have now found a new method of preparing allylic aromatic compounds such as allyl-3,5-dinitrobenzene which can give the required product in high yield from commercially available and less hazardous starting materials without the use of metal reagents or catalysts. The procedure is more convenient in that only a single process step is required and the reaction time is short. The method is therefore suitable for use for large scale manufacture.

The invention thus provides a process for the preparation of an allylic aromatic compound having at least one electron withdrawing substituent on the aromatic ring in which a correspondingly ring-substituted aromatic amine is reacted first with a nitrite and then with an allylic olefin having an eliminatable terminal substituent.

The aromatic amine starting material is preferably substituted by one or two electron withdrawing groups and may have the formula R-NH₂ where:
R is a phenyl or heterocyclic aromatic group optionally substituted by one or more (e.g. one or two) of the following groups: OH, OX, OCN, OCOX, OCONH₂, OSO₂H, OSO₂X, OSiX₃, OPOX², X, CHO, COX, COOH, COOX, CONHZ, CONX², CN, NO₂, NCO, NCS, NC, NHZ, NX², NZOH, NZCHO, NZCOX, NZCO₂X, NZCONH₂, NZSO₂X, SH, SX, SOX, SO₂X, SO₂NHZ, SO₂NX², SCN, F, Cl, Br, B(OH)₂, B(OX)₂, or PO(OX)₂;
X is a phenyl or C₁-C₁₀ alkyl group optionally substituted by F, Cl, Br and/or substituents containing F, Cl, Br, O, S or N (such as halo-C₁₋₃ alkyl, C₂₋₆ alkanoyloxy, C₁₋₆ alkoxy, OH, OCN, OCONH₂, OSO₂H, CHO, COOH, CONH₂, NC, NO₂, NCO, NCS, CN, NH₂, SH, SO₂NH₂ or SCN);
X² represents two X groups or a C₃₋₈ cycloalkyl group optionally substituted as defined for X, for example by F, Cl and/or Br; and
Z is H or X.

When R is a heterocyclic group, it may contain O, N or S as the heteroatom and may be monocyclic or bicyclic.

The aromatic amine preferably has the formula (1) 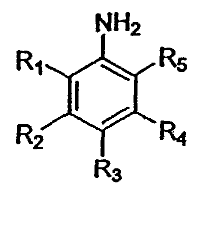 where:
R₁, R₃ and R₅ are independently H or NO₂, and
R₂ and R₄ are independently H, NO₂, COOH, COOMe, COOEt, CONH₂, CONHPh, COMe, CN or CF₃, provided that at least one (and preferably at least two) of R₁-R₅ is other than H.

Examples of suitable amines are 3-nitroaniline, 4-nitroaniline, 2,4-dinitroaniline, 2,6-dinitroaniline, 3,5-dinitroaniline, 3-acetyl-5-nitroaniline, 3,5-diacetylaniline, 3-carboxymethyl-5-nitroaniline, 3-carboxyethyl-5-nitroaniline, 3,5-dicarboxyethylaniline, 3,5-dicarboxymethylaniline and 3,5-bisaminocarbonylaniline.

A preferred amine starting material is 3,5-dinitroaniline.

The nitrite used may be an organic nitrite such as a C₁₋₆ alkyl nitrite, preferably t-butylnitrite, or an aromatic nitrite.

The allylic olefin reagent may be a compound of the formula R^{a}L where R^{a} is a 2,3-alkenyl group (such as a C₃₋₆ alkenyl or C₅₋₇ cycloalkenyl group which may be optionally substituted) and L is an eliminatable substituent.

The allylic reagent may for example have the formula (2) 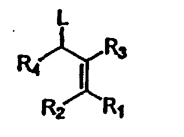 where
L is Br, I, SR^{b}, SnR^{b}₃, SiR^{b}₃, Si(TRS)₃, SSi(TRS)₃, SSnR^{b}₃, SO₂R^{b}, SO₂CF₃, SePh or OPO(OPh)₂ (where TRS is trialkylsilyl and R^{b} is C₁₋₆ alkyl, C₂₋₆ alkenyl or aryl such as phenyl or tolyl), and
R₁-R₄, independently, is each a hydrogen atom or a phenyl or C₁-C₁₀ alkyl group optionally substituted by F, Cl, Br and/or substituents containing F, Cl, Br, O, S, N such as given above for X. R¹ and R³ may also be Br or -COOH

Examples of such compounds are 3-bromopropene, 3-bromo-2-methylpropene, 2-bromomethylacrylic acid, 2-acetoxymethyl-3-bromopropene, 1,4-dibromo-2-butene, 1-acetoxy-4-bromo-2-butene, 1-hydroxy-2-bromo-3-butene, 1-acetoxy-2-bromo-3-butene, allyldisulfide, allylsulfide, allylmethylsulfide, allyliodide and allyl bromide.

It is believed that in the initial stage of the reaction, the nitrite diazotises the aromatic amine and forms an aryl radical which then reacts with the allylic reagent R^{a}L to produce the required allylic aromatic compound by elimination of the substituent L.

In reactions where the allylic reagent and/or the nitrite are liquid it is not necessary to use a solvent, but a polar solvent such as acetonitrile or dimethylsulphoxide can be used if required. The reaction temperature is preferably kept relatively low (e.g. 5-15°C), and if desired can then be increased to 20-40°C, conveniently room temperature, to ensure that the reaction is completed.

As indicated above, the process of the invention is particularly suitable for the preparation of 1-allyl-3,5-dinitrobenzene, which is a new compound and is a further aspect of the invention. The preparation is preferably conducted by reaction of 3,5-dinitroaniline with t-butylnitrite and allyl bromide, using the allyl bromide and the nitrite as the reaction solvent. The reaction can be completed within 2-2.5 hours to give a yield of over 90% of the required compound, and is suitable for large scale use. This compares very favourably with the use of toxic and expensive tin catalysts. Thus, direct allylation of 3,5-dinitroiodobenzene with tributylallyltin in the presence of a palladium complex catalyst only gave a yield of 20% after a reaction time of one week.

The following examples illustrate the invention.

### EXAMPLE 1

### 1-Allyl-3,5-dinitrobenzene

To a solution of t-butylnitrite (84.6 ml, 0.71 mol) and allylbromide (530 ml, 6.15 mol) in 25 ml CH₃CN, 3,5-dinitroaniline (75 g, 0.41 mol) was added spoonwise, so that the temperature was maintained between 11 and 15°C. During the addition of the final 1/2 of 3,5-dinitroaniline, more t-butylnitrite (21 ml, 0.18 mol) was added. The reaction mixture was then stirred at room temperature for 1 h. Excess t-butylnitrite, allylbromide and CH₃CN was distilled from the reaction mixture at reduced pressure and the residue was dissolved in toluene (500 ml). The solution was filtered through two Al₂O₃ pads. The toluene was distilled off at reduced pressure and to the remaining residue 3,3,5-trimethylpentane (200 ml) was added. The mixture was heated to 60°C and stirred for 1/2 h and then cooled to -60°C. The pentane phase was decanted and the mixture was crystallized from 2,2,4-trimethylpentane at -50°C. The crystals were then triturated twice with 2,2,4-trimethylpentane at -50°C. The remaining pentane was then removed at reduced pressure, to give 78.7 g (93%) of 3,5-dinitroallylbenzene as yellow oil containing approximately 4% of 3,5-dinitrobromo benzene.
¹H-NMR (CDCl₃, 300 MHz) δ ppm: 8.90 (t, J = 2.0 Hz, 1H), 8.39 (d, J = 2.0 Hz, 2H), 6.04-5.90 (m, 1H), 5.30-5.18 (m, 2H), 3.62 (d, J = 6.7, 2H). ¹³C-NMR (CDCl₃, 75.43 MHz) δ ppm: 148.5, 144.5, 133.9, 128.8, 119.1, 116.8, 39.4.

### EXAMPLE 2

### 3-Acetyl-5-nitroallylbenzene

To a solution of t-butylnitrite (0.24 ml, 2.1 mmol) and allylbromide (1.8 ml, 25.2 mmol) in 3 ml CH₃CN 3-acetyl-5-nitroaniline (0.25 g, 1.4 mmol) was added, so that the temperature was maintained between 20 and 25°C, and the mixture was stirred for 10 min at room temperature. The volatile material in the reaction solution was then removed at reduced pressure to give 3-acetyl-5-nitroallylbenzene. ¹H-NMR (CDCl₃, 300 MHz) δ ppm: 8.61 (s, 1H), 8.25 (s, 1H), 8.10 (s, 1H), 6.03-5.88 (m, 1H), 5.23-5.11 (m, 2H), 3.55 (d, 2H).

### EXAMPLE 3

### 1-(2-Methyl-2-propen-1-yl)-3,5-dinitrobenzene

To a solution of t-butylnitrite (0.13 ml, 1.1 mmol) and 3-bromo-2-methylpropene (0.82 ml, 8.25 mmol) in 1 ml CH₃CN, 3,5-dinitroaniline (0.10 g, 0.55 mmol) was added at room temperature. The solution was stirred for 17 h at room temperature and then isopropylether was added. The suspension was filtered through a pad of Al₂O₃. The volatile material in the solution was removed at reduced pressure to give the title compound. ¹H-NMR (CDCl₃, 300 MHz) δ ppm: 8.92 (t, J = 2.1 Hz, 1H), 8.40 (d, J = 2.1 Hz, 2H), 5.00 (s, 1H), 4.82 (s, 1H), 3.55 (s, 2H), 1.72 (s, 3H). ¹³C-NMR (CDCl₃, 75.43 MHz) δ ppm: 148.4, 144.2, 142.0, 129.0, 116.9, 114.8, 43.9, 22.0.

### EXAMPLE 4

### 1 - (2 -Acetoxymethyl -2 -propen -1-yl ) -3,5 -dinitrobenzene

To a solution of t-butylnitrite (0.47 ml, 4.0 mmol) and 2-acetoxymethyl-3-bromopropene (1.93 g, 10.0 mmol) in 1 ml CH₃CN, 3,5-dinitroaniline (0.37 g, 2.0 mmol) was added, while maintaining the temperature between 18 and 24°C. The mixture was stirred for 1 h at room temperature. The volatile material in the solution was removed at reduced pressure. Flash chromatography (heptane/ethylacetate) gave 0.42 g 1-(2-acetoxymethyl-2-propenyl)-3,5-dinitrobenzene. ¹H-NMR (CDCl₃, 300 MHz) δ ppm: 8.94 (t, J = 2.1 Hz, 1H), 8.42 (d, J = 2.1 Hz, 2H), 5.33 (s, 1H), 5.05 (s, 1H), 4.52 (s, 2H), 3.64 (s, 2H), 2.08 (s, 3H). ¹³C-NMR (CDCl₃, 75.43 MHz) δ ppm: 170.4, 148.5, 143.1, 140.7, 129.1, 117.5, 117.2, 65.9, 39.3, 20.7.

### EXAMPLE 5

### Different leaving groups (L)

To a solution of t-butylnitrite (119 µl, 1.0 mmol) and the allylic reagent (CH₂=CHCH₂L, 5 mmol) in CH₃CN (0.5 ml), 3,5-dinitroaniline (92 mg, 0.5 mmol) was added during 10 minutes, while maintaining the temperature of the reaction mixture at 12-14⁰C. The reaction mixture was then stirred at 22⁰C for one hour. 2-Nitrobenzylalcohol (50 mg) was added as an internal standard. The yield was determined by HPLC.

| L | Yield (%) |
|---|---|
| Br | 74 |
| SO₂Ph | 51 |
| SMe | 28 |
| S(allyl) | 34 |
| SPh | 61 |
| SiMe₃ | 25 |
| I | 18 |

### EXAMPLE 6

### Allyl-2,4-dinitrobenzene

To a solution of t-butylnitrite (119 µl, 1.0 mmol) and allyl bromide (0.65 ml, 7.5 mmol) in CH₃CN (5 ml), 2,4-dinitroaniline (92 mg, 0.5 mmol) was added during 10 minutes, while maintaining the temperature of the reaction mixture at 30-35⁰C. Extra t-butylnitrite (119 µl, 1.0 mmol) was added to the reaction mixture which then was stirred at 35⁰C for one hour. Despite larger amounts of t-butylnitrite and higher reaction temperature, approximately 10% of the aniline was still left accordingly to HPLC. The volatile material in the reaction mixture was then removed at reduced pressure. Chromatography (heptane-ethyl acetate 23:2) gave 60 mg of a 87:13 mixture of allyl-2,4-dinitrobenzene and 2,4-dinitrobromobenzene as a yellow oil, which corresponds to 49 % yield of allyl-2,4-dinitrobenzene. ¹H NMR (CDCl₃, 400 MHz) δ 8.77, (d, 1H, J = 2.4 Hz), 8.39, (dd, 1H, J = 8.5, 2.1 Hz), 7.64, (d, 1H, J = 8.5 Hz), 6.01-5.90, (m, 1H), 5.24-5.12, (m, 2H), 3.80, (d, 2H, J = 6.5 Hz).

### EXAMPLE 8

### Allyl-2-cyano-4-nitrobenzene

To a solution of t-butylnitrite (535 µl, 4.5 mmol) and allyl bromide (3.9 ml, 45.0 mmol) in CH₃CN (3 ml), 2-cyano-4-nitroaniline (489 mg, 3.0 mmol) was added during 40 minutes, while maintaining the temperature of the reaction mixture at 23-27⁰C. At the end of the addition of the aniline, extra t-butylnitrite (180 µl, 1.5 mmol) was added to the reaction mixture which then was stirred at 26⁰C for one hour. The volatile material in the reaction mixture was removed at reduced pressure. Column chromatography (heptane-ethyl acetate 23:2) gave 367 mg (65%) of allyl-2-cyano-4-nitrobenzene as an orange oil. ¹H NMR (CDCl₃, 400 MHz) δ 8.51, (d, 1H, J = 2.4 Hz), 8.39, (dd, 1H, J = 8.6, 2.4 Hz), 7.58, (dd, 1H, J = 8.6, 0.5 Hz), 6.00-5.90, (m, 1H), 5.29-5.17, (m, 2H), 3.74, (d, 2H, J = 6.6 Hz); ¹³C NMR (CDCl₃, 100 MHz) δ 151.3, 146.8, 133.5, 131.4, 128.4,127.9, 119.6, 116.2, 114.4, 38.9; Anal HRMS Calcd. for C₁₀H₈N₂O₂ (M) : 188.0585. Found: 188.0585.

### EXAMPLE 9

### Allyl-4-nitro-2-trifluoromethylbenzene

To a solution of t-butylnitrite (535 µl, 4.5 mmol) and allyl bromide (3.9 ml, 45.0 mmol) in CH₃CN (3 ml), 4-nitro-2-trifluoromethylaniline (618 mg, 3.0 mmol) was added during 20 minutes while maintaining the temperature of the reaction mixture at 18-19⁰C. At the end of the addition of the aniline, extra t-butylnitrite (180 µl, 1.5 mmol) was added to the reaction mixture which then was stirred at 25⁰C for one hour. The volatile material in the reaction mixture was removed at reduced pressure. Column chromatography (heptane-ethyl acetate 49:1) gave 587 mg (85%) of allyl-4-nitro-2-trifluoromethylbenzene as a light yellow oil. ¹H NMR (CDCl₃, 400 MHz) δ 8.53, (d, 1H, J = 2.4 Hz), 8.35, (dd, 1H, J = 8.5, 2.4 Hz), 7.59, (d, 1H, J = 8.5 Hz), 6.00-5.88, (m, 1H), 5.24-5.12, (m, 2H), 3.68, (d, 2H, J = 6.5 Hz); ¹³C NMK (CDCl₃, 100 MHz) δ 146.8, 146.6, 134.9, 133.0, 130.3, (q, J = 32.2 Hz), 127.7, 126.9, 124.9, 122.2, 122.1, (q, J = 6.0 Hz), 118.7, 37.0, (m); Anal HRMS Calcd. for C₁₀H₈F₃NO₂ (M) : 231.0507. Found: 208.0515.

### EXAMPLE 10

### Allyl-4-nitro-3-trifluoromethylbenzene

To a solution of t-butylnitrite (535 µl, 4.5 mmol) and allyl bromide (3.9 ml, 45.0 mmol) in CH₃CN (3 ml), 4-nitro-3-trifluoromethylaniline (618 mg, 3.0 mmol) was added during 20 minutes, while maintaining the temperature of the reaction mixture between 13-15⁰C. At the end of the addition of the aniline, extra t-butylnitrite (180 µl, 1.5 mmol) was added to the reaction mixture which then was stirred at 25⁰C for one hour. The volatile material in the reaction mixture was removed at reduced pressure. Column chromatography (heptane-ethyl acetate 97:3) gave 430 mg (62%) of allyl-4-nitro-3-trifluoromethylbenzene as a light yellow oil. ¹H NMR (CDCl₃, 400 MHz) δ 7.86, (d, 1H, J = 8.3 Hz), 7.65, (d, 1H, J = 1.2 Hz), 7.55, (dd, 1H, J = 8.3, 1.4 Hz), 6.00-5.89, (m, 1H), 5.26-5.14, (m, 2H) , 3.54, (d, 2H, J = 6.7 Hz); ¹³C NMR (CDCl₃, 100 MHz) δ 146.5, 135.0, 133.3, 128.5, (q, J = 5.0 Hz), 125.8, 124.4, 124.1, 123.8, 121.1, 118.8, 40.0; Anal HRMS Calcd. for C₁₀H₈F₃NO₂ (M): 231.0507. Found: 208.0499.

### EXAMPLE 11

### 2-Allyl-5-nitrobenzophenone

To a solution of t-butylnitrite (535 µl, 4.5 mmol) and allyl bromide (3.9 ml, 45.0 mmol) in CH₃CN (3 ml), 2-amino-5-nitrobenzophenone (727 mg, 3.0 mmol) was added during 20 minutes while maintaining the temperature of the reaction mixture at 28-32⁰C. At the end of the addition of the aniline, extra t-butylnitrite (360 µl, 3 mmol) was added to the reaction mixture which then was stirred at 25⁰C for one hour. The volatile material in the reaction mixture was removed at reduced pressure. Column chromatography (heptane-ethyl acetate 23:2) gave 370 mg (46%) of 2-allyl-5-nitrobenzophenone as a light yellow oil. ¹H NMR (CDCl₃, 300 MHz) δ 8.29, (dd, 1H, J = 8.6, 2.5 Hz), 8.18, (d, 1H, J = 2.4 Hz), 7.81-7.74, (m, 2H), 7.69-7.60, (m, 1H), 7.57-7.45, (m, 3H), 5.93-5.88, (m, 1H), 5.09-5.95, (m, 2H), 3.53, (m, 2H, J = 6.6 Hz).

### EXAMPLE 12

### Ethyl 3-allyl-5-nitrobenzoate

To a solution of t-butylnitrite (535 µl, 4.5 mmol) and allyl bromide (3.9 ml, 45.0 mmol) in CH₃CN (3 ml), ethyl 3-amino-5-nitrobenzoate (631 mg, 3.0 mmol) was added during 20 minutes while maintaining the temperature of the reaction mixture at 10-12⁰C. At the end of the addition of the aniline, extra t-butylnitrite (180 µl, 1.5 mmol) was added to the reaction mixture which then was stirred at 25⁰C for one hour. The volatile material in the reaction mixture was removed at reduced pressure. Column chromatography (heptane-ethyl acetate 47:3) gave 470 mg (67%) of ethyl 3-allyl-5-nitrobenzoate as a light yellow oil, which crystallized upon standing overnight in the refrigerator. ¹H NMR (CDCl₃, 400 MHz) δ 8.70, (m, 1H), 8.24, (m, 1H), 8.20, (m, 1H), 6.03-5.91, (m, 1H), 5.24-5.13, (m, 2H), 4.44, (q, 2H, J = 7.1 Hz), 3.56, (d, 2H, J = 6.5 Hz), 1.44, (t, 3H, J = 7.1 Hz); ¹³C NMR (CDCl₃, 100 MHz) δ 165.0, 148.8, 143.1, 135.9, 135.5, 132.6, 127.7, 122.9, 118.4, 62.3, 39.9, 14.7; Anal HRMS Calcd. for C₁₂H₁₃NO₄ (M): 235.0845. Found: 235.0846.

### EXAMPLE 13

### Allyl-2-chloro-4-nitrobenzene

To a solution of t-butylnitrite (535 µl, 4.5 mmol) and allyl bromide (3.9 ml, 45.0 mmol) in CH₃CN (3 ml), 2-chloro-4-nitroaniline (518 mg, 3.0 mmol) was added during 20 minutes, while maintaining the temperature of the reaction mixture at 11-13⁰C. At the end of the addition of the aniline, extra t-butylnitrite (180 µl, 1.5 mmol) was added to the reaction mixture which then was stirred at 23⁰C for one hour. The volatile material in the reaction mixture was then removed at reduced pressure. Column chromatography (heptane-ethyl acetate 97:3) gave 497 mg (84%) allyl-2-chloro-4-nitrobenzene as a light yellow oil containing 5% of 2-chloro-4-nitrobromobenzene. This corresponds to 79% yield of allyl-2-chloro-4-nitrobenzene. ¹H NMR (CDCl₃, 400 MHz) δ 8.25, (d, 1H, J = 2.4 Hz), 8.08, (dd, 1H, J = 8.5, 2.3 Hz), 7.42, (d, 1H, J = 8.5 Hz), 6.01-5.89, (m, 1H), 5.23-5.10, (m, 2H), 3.59, (d, 2H, J = 6.5 Hz) ; ¹³C NMR (CDCl₃, 100 MHz) δ 147.3, 145.9, 135.3, 134.0, 131.2, 125.0, 122.2, 118.5, 38.0; Anal HRMS Calcd. for C₉H₈ClNO₂ (M): 197.0244. Found: 197.0247.

### EXAMPLE 14

### Allyl-2-bromo-5-nitrobenzene

To a solution of t-butylnitrite (535 µl, 4.5 mmol) and allyl bromide (3.9 ml, 45.0 mmol) in CH₃CN (3 ml), 2-bromo-5-nitroaniline (651 mg, 3.0 mmol) was added during 20 minutes, while maintaining the temperature of the reaction mixture at 35-40⁰C. At the end of the addition of the aniline, extra t-butylnitrite (180 µl, 1.5 mmol) was added to the reaction mixture which then was stirred at 23⁰C for one hour. The volatile material in the reaction mixture was removed at reduced pressure. Column chromatography (heptane-ethyl acetate 49:1) gave 568 mg (78%) allyl-2-bromo-5-nitrobenzene as a light yellow oil containing 7% of 3,4-dibromo-nitrobenzene, which crystallized upon standing overnight in the refrigerator. This corresponds to 72% yield of allyl-2-bromo-5-nitrobenzene. The allyl-2-bromo-5-nitrobenzene could be purified by crystallization from pentane. ¹H NMR (CDCl₃, 400 MHz) δ 8.10, (d, 1H, J = 2.7 Hz), 7.95, (dd, 1H, J = 8.7, 2.7 Hz), 7.74, (d, 1H, J = 8.7 Hz), 6.03-5.92, (m, 1H), 5.26-5.13, (m, 2H), 3.60, (m, 2H, J = 6.5 Hz); ¹³C NMR (CDCl₃, 100 MHz) δ 147.7, 142.0, 134.1, 134.1, 132.3, 125.3, 122.9, 118.8, 40.5; Anal HRMS Calcd. for C₉H₈BrNO₂ (M): 240.9738. Found: 240.9739.

### EXAMPLE 15

### Allyl -4 -methoxy -3 -nitrobenzene

To a solution of t-butylnitrite (535 µl, 4.5 mmol) and allyl bromide (3.9 ml, 45.0 mmol) in CH₃CN (3 ml), 4-methoxy-3-nitroaniline (504 mg, 3.0 mmol) was added during 20 minutes, while maintaining the temperature of the reaction mixture at 35-40⁰C. At the end of the addition of the aniline, extra t-butylnitrite (180 µl, 1.5 mmol) was added to the reaction mixture which then was stirred at 40⁰C for one hour. The volatile material in the reaction mixture was removed at reduced pressure. The crude product was dissolved in ethyl acetate-heptane (1:2, 20 ml) and filtered through a pad of silica. The solvent was removed at reduced pressure. Column chromatography (heptane-ethyl acetate 97:3) gave 230 mg (40%) allyl-4-methoxy-3-nitrobenzene as a yellow oil. ¹H NMR (CDCl₃, 400 MHz) δ 7.45, (d, 1H, J = 2.7 Hz), 7.27, (d, 1H, J = 8.6 Hz), 7.10, (dd, 1H, J = 8.6, 2.7 Hz), 6.02-5.90, (m, 1H), 5.12-5.02, (m, 2H), 3.86, (s, 3H), 3.62, (m, 2H, J = 6.4 Hz); ¹³C NMR (CDCl₃, 100 MHz) δ 158.8, 150.0, 135.9, 133.2, 127.2, 120.2, 117.1, 109.6, 56.2, 36.8; Anal HRMS Calcd. for C₁₀H₁₁NO₃ (M) : 193.0739. Found: 193.0743

### EXAMPLE 16

### Allyl-2-methyl-3-nitrobenzene

To a solution of t-butylnitrite (535 µl, 4.5 mmol) and allyl bromide (3.9 ml, 45.0 mmol) in CH₃CN (3 ml), 2-methyl-3-nitroaniline (651 mg, 3.0 mmol) was added during 20 minutes, while maintaining the temperature of the reaction mixture at 32-35⁰C. At the end of the addition of the aniline, extra t-butylnitrite (180 µl, 1.5 mmol) was added to the reaction mixture which then was stirred at 23⁰C for one hour. The volatile material in the reaction mixture was removed at reduced pressure. Column chromatography (heptane-ethyl acetate 49:1) gave 370 mg of a 25:6:69 mixture of 2-methyl-3-nitrobromobenzene, monobromo-1-allyl-2-methyl-3-nitrobenzene and of allyl-2-methyl-3-nitrobenzene. (The byproducts have been identified with HRMS). This corresponds to 43% yield of allyl-2-methyl-3-nitrobenzene. ¹H NMR (CDCl₃, 300 MHz) δ 7.63, (d, 1H, J = 8.2 Hz), 7.38, (d, 1H, J = 7.4 Hz), 7.26, (t, 1H, J = 7.8 Hz), 6.01-5.86, (m, 1H), 5.17-4.93, (m, 2H), 3.47, (m, 2H, J = 6.0 Hz), 2.39, (s, 3H); Anal HRMS Calcd. for C₁₀H₁₁NO₂ (M): 177.0790. Found: 177.0790.

### EXAMPLE 17

### Allyl-3,5-dichlorobenzene

To a solution of t-butylnitrite (535 µl, 4.5 mmol) and allyl bromide (3.9 ml, 45.0 mmol) in CH₃CN (3 ml), 3,5-dichloroaniline (486 mg, 3.0 mmol) was added during 20 minutes, while maintaining the temperature of the reaction mixture at 30-35⁰C. At the end of the addition of the aniline, extra t-butylnitrite (180 µl, 1.5 mmol) was added to the reaction mixture which then was stirred at 23⁰C for one hour. The volatile material in the reaction mixture was removed at reduced pressure. Column chromatography (pentane) gave 374 mg of a 17:4:79 mixture of allyl-4-bromo-3,5-dichlorobenzene, bromo-3,5-dichlorobenzene and of allyl-3,5-dichlorobenzene. (The by-products have been identified with HRMS and ¹H NMR). This corresponds to 48% yield of allyl-3,5-dichlorobenzene. ¹H NMR (CDCl₃, 400 MHz) δ 7.22, (m, 1H, J = 1.9, 0.3 Hz), 7.09, (m, 2H, J = 1.9 Hz), 5.97-5.86, (m, 1H), 5.18-5.09, (m, 2H), 3.35, (d, 2H, J = 6.7 Hz); ¹³C NMR (CDCl₃, 100 MHz) δ 143.8, 136.1, 135.2, 127.6, 126.8, 117.7, 39.9; Anal HRMS Calcd. for C₉H₈Cl₂ (M): 186.0003. Found: 186.0003.

### EXAMPLE 18

### Allyl-3,5-dibromobenzene

To a solution of t-butylnitrite (59.5 µl, 0.5 mmol) and allyl bromide (0.33 ml, 3.75 mmol) in CH₃CN (0.25 ml), 3,5-dibromoaniline (63 mg, 0.25 mmol) was added during 5 minutes, while maintaining the temperature at 30-35⁰C. The reaction mixture was then stirred at 23⁰C for one hour. The volatile material in the reaction mixture was removed at reduced pressure. Column chromatography (pentane) gave 40 mg of a 12:15:73 mixture of allyl-4-bromo-3,5-dibromobenzene, 1,3,5-tribromobenzene and allyl-3,5-dibromobenzene. (The byproducts have been identified with HRMS and ¹H NMR). This corresponds to 39% yield of allyl-3,5-dibromobenzene. ¹H NMR (CDCl₃, 300 MHz) 5 7.51, (m, 1H, J = 1.8, 0.4 Hz), 7.27, (m, 2H, J = 1.8 Hz), 5.96-5.82, (m, 1H), 5.17-5.07, (m, 2H), 3.33, (d, 2H, J = 6.5 Hz); Anal HRMS Calcd. for C₉H₈Br₂ (M): 273.8993. Found: 273.8984.

### EXAMPLE 19

### 4-Allylbromobenzene

To a solution of t-butylnitrite (535 µl, 4.5 mmol) and allyl bromide (3.9 ml, 45.0 mmol) in CH₃CN (3 ml), 4-bromoaniline (516 mg, 3.0 mmol) was added during 20 minutes, while maintaining the temperature of the reaction mixture at 30-35⁰C. At the end of the addition of the aniline, extra t-butylnitrite (180 µl, 1.5 mmol) was added to the reaction mixture which then was stirred at 30⁰C for one hour. The volatile material in the reaction mixture was removed at reduced pressure. Column chromatography (pentane) gave 200 mg (34%) of 4-allylbromobenzene as a clear oil. ¹H NMR (CDCl₃, 400 MHz) δ 7.43, (m, 2H, J = 8.3 Hz), 7.08, (m, 2H, J = 8.3 Hz), 6.00-5.89, (m, 1H), 5.12-5.05, (m, 2H), 3.35, (d, 2H, J = 6.7 Hz); ¹³C NMR (CDCl₃, 100 MHz) δ 139.4, 137.2, 131.9, 130.8, 120.3, 116.7, 40.0; Anal HRMS Calcd. for C₉H₉Br (M): 195.9888. Found: 195.9887.

### EXAMPLE 20

### 4-Allylbenzonitrile

To a solution of t-butylnitrite (535 µl, 4.5 mmol) and allyl bromide (3.9 ml, 45.0 mmol) in CH₃CN (3 ml), 4-aminobenzonitrile (354 mg, 3.0 mmol) was added during 20 minutes, while maintaining the temperature of the reaction mixture at 42-44⁰C. At the end of the addition of the aniline, extra t-butylnitrite (180 µl, 1.5 mmol) was added to the reaction mixture which then was stirred at 50⁰C for three hours during which extra t-butylnitrite (180 µl, 1.5 mmol) was added. The volatile material in the reaction mixture was removed at reduced pressure. Heptane-ethyl acetate (10 ml, 1:1) was then added to the crude product and the mixture was filtered. Column chromatography (heptane-ethyl acetate 49:1) of the concentrated filtrate gave 160 mg (37%) 4-allylbenzonitrile as a clear oil containing 16% of 4-bromobenzonitrile. This corresponds to 30% yield of 4-allylbenzonitrile. ¹H NMR (CDCl₃, 400 MHz) δ 7.60, (m, 2H, J = 8.3 Hz), 7.31, (m, 2H, J = 8.3 Hz), 5.99-5.88, (m, 1H), 5.18-5.08, (m, 2H), 3.46, (d, 2H, J = 6.7 Hz); Anal HRMS Calcd. for C₁₀H₉N (M): 143.0735. Found: 143.0734.

### EXAMPLE 21

### 3-Allylnitrobenzene

To a solution of t-butylnitrite (535 µl, 4.5 mmol) and allyl bromide (3.9 ml, 45.0 mmol) in CH₃CN (30 ml), 3-nitroaniline (414 mg, 3.0 mmol) was added during 20 minutes, while maintaining the temperature of the reaction mixture at 34-36⁰C. At the end of the addition of the aniline, extra t-butylnitrite (180 µl, 1.5 mmol) was added to the reaction mixture which then was stirred at 35⁰C for one hour. The volatile material in the reaction mixture was removed at reduced pressure. Column chromatography (heptane-ethyl acetate 47:3) gave 420 mg of 3-allylnitrobenzene as a light yellow oil containing 36% of 3-bromonitrobenzene. This corresponds to 50% yield of 3-allylnitrobenzene. ¹H NMR (CDCl₃, 400 MHz) δ 8.10-8.08, (m, 1H), 8.08-8.05, (m, 1H), 7.56-7.51, (m, 1H), 7.50-7.44, (m, 1H), 6.02-5.91, (m, 1H), 5.20-5.10, (m, 2H), 3.50, (d, 2H, J = 6.7 Hz); ¹³C NMR (CDCl₃, 100 MHz) δ 148.8, 142.5, 136.1, 135.3, 129.7, 123.9, 121.8, 117.8, 40.1.

### EXAMPLE 22

### 4-Allylnitrobenzene

To a solution of t-butylnitrite (535 µl, 4.5 mmol) and allyl bromide (3.9 ml, 45.0 mmol) in CH₃CN (30 ml), 4-nitroaniline (414 mg, 3.0 mmol) was added during 20 minutes, while maintaining the temperature of the reaction mixture at 34-36⁰C. At the end of the addition of the aniline, extra t-butylnitrite (180 µl, 1.5 mmol) was added to the reaction mixture which then was stirred at 35⁰C for one hour. The volatile material in the reaction mixture was removed at reduced pressure. Column chromatography (heptane-ethyl acetate 23:2) gave 330 mg of 4-allylnitrobenzene as a light yellow oil containing 15% of 4-bromonitrobenzene. This corresponds to 55% yield of 4-allylnitrobenzene. ¹H NMR (CDCl₃, 300 MHz) δ 8.16, (m, 2H, J = 8.6 Hz), 7.35, (m, 2H, J = 8.6 Hz), 6.02-5.88, (m, 1H), 5.19-5.08, (m, 2H), 3.49, (d, 2H, J = 6.6 Hz).

### EXAMPLE 23

### 2-Allyl-5-nitropyridine

To a solution of t-butylnitrite (535 µl, 4.5 mmol) and allyl bromide (3.9 ml, 45.0 mmol) in CH₃CN (4 ml), 2-amino-5-nitropyridine (417 mg, 3.0 mmol) was added during 20 minutes, while maintaining the temperature of the reaction mixture at 35-38⁰C. At the end of the addition of the aniline, extra t-butylnitrite (180 µl, 1.5 mmol) was added to the reaction mixture which then was stirred at 37⁰C for one hour. The volatile material in the reaction mixture was removed at reduced pressure to give a crude product containing 2-allyl-5-nitropyridine. ¹H NMR (CDCl₃, 300 MHz) δ 9.37, (d, 1H, J = 2.8 Hz), 8.41, (dd, 1H, J = 8.6, 2.7 Hz), 7.39, (d, 1H, J = 8.4 Hz), 6.12-5.97, (m, 1H), 5.27-5.18, (m, 2H), 3.72, (m, 2H, J = 6.8, 1.4 Hz).

### EXAMPLE 24

### 3,5-Dinitro-1-(2-phenyl-2-propenyl)benzene

To a solution of t-butylnitrite (119 µl, 1.0 mmol) and 2-phenyl-3-brompropene (0.99 g, 5.0 mmol) in CH₃CN (0.5 ml) was 3,5-dinitroaniline (92 mg, 0.5 mmol) added during 10 minutes, while maintaining the temperature of the reaction mixture at 23-28⁰C. At the end of the addition of the aniline extra t-butylnitrite (119 µl, 1.0 mmol) was added to the reaction mixture which then was heated to 35⁰C for 5 minutes. The reaction mixture was stirred at 22⁰C for one hour. The volatile material in the reaction mixture was removed at reduced pressure. Column chromatography (heptane-ethyl acetate 23:2) succeeded by crystallization from heptane gave 50 mg (35%) of 3,5-dinitro-1-(2-phenyl-2-propenyl)benzene as bright yellow needles. ¹H NMR (CDCl₃, 400 MHz) δ 8.88, (t, 1H, J = 2.1 Hz), 8.42, (d, 2H, J = 2.1 Hz), 7.42-7.37, (m, 2H), 7.36-7.24, (m, 3H), 5.65, (s, 1H), 5.22, (d, 1H, J = 0.8 Hz), 4.08, (s, 2H).

### EXAMPLE 25

### 3,5 -Dinitro-1-(2 -bromo -2-propenyl)benzene

To a solution of t-butylnitrite (535 µl, 4.5 mmol) and allyl bromide (3.9 ml, 45.0 mmol) in CH₃CN (3 ml), 3,5-dinitroaniline (549 mg, 3.0 mmol) was added during 20 minutes, while maintaining the temperature of the reaction mixture at 11-15°C. At the end of the addition of the aniline extra t-butylnitrite (180 µl, 1.5 mmol) was added to the reaction mixture which then was stirred at 22⁰C for one hour. The volatile material in the reaction mixture was removed at reduced pressure. Column chromatography (heptane-ethyl acetate 10:1) gave 800 mg (93%) 3,5-dinitro-1-(2-bromo-2-propenyl)benzene as a yellow oil. ¹H NMR (CDCl₃, 400 MHz) δ 8.98, (t, 1H, J = 2.1 Hz), 8.47, (d, 2H, J = 2.1 Hz), 5.89, (m, 1H), 5.71, (d, 1H, J = 2.1 Hz), 4.00, (s, 2H); ¹³C NMR (CDCl₃, 100 MHz) δ 149.05, 142.1, 129.6, 129.3, 121.4, 118.2, 47.3.

### EXAMPLE 26

### 3-(3,5-Dinitrophenyl)cyclohexene

To a solution of t-butylnitrite (119 µl, 1.0 mmol) and 3-bromocyclohexene (860 µl, 7.5 mmol) in CH₃CN (0.5 ml), 3,5-dinitroaniline (92 mg, 0.5 mmol) was added during 10 minutes, while maintaining the temperature of the reaction mixture at 23-30⁰C. After stirring for one hour at room temperature the volatile material in the reaction mixture was removed at reduced pressure. Column chromatography (heptane-ethyl acetate 23:2) followed by preparative HPLC gave 15 mg (12%) of 3-(3,5-dinitrophenyl)cyclohexene as bright yellow crystals. -H NMR (CDCl₃, 300 MHz) δ 8.90, (t, 1H, J = 2.1 Hz), 8.42, (d, 2H, J = 2.1 Hz), 6.19-6.06, (m, 1H), 5.74-5.66, (m, 1H), 3.67, (m, 1H), 2.25-2.07, (m, 3H), 1.80-1.51, (m, 3H).

### EXAMPLE 27

### 2-(3,5-Dinitrobenzyl)acrylic acid

To a solution of t-butylnitrite (535 µl, 4.5 mmol) and 2-(bromomethyl)acrylic acid (7.42 g, 45.0 mmol) in CH₃CN (10 ml), 3,5-dinitroaniline (549 mg, 3.0 mmol) was added during 20 minutes, while maintaining the temperature of the reaction mixture at 15-20⁰C. At the end of the addition of the aniline extra t-butylnitrite (180 µl, 1.5 mmol) was added to the reaction mixture. After heating to 35⁰C for 5 minutes, the mixture was stirred at room temperature for one hour. The volatile material in the reaction mixture was then removed at reduced pressure. The remaining crystalline residue was washed with heptane (5x50 ml) to remove unreacted 2-(bromomethyl)acrylic acid. Column chromatography (heptane-ethyl acetate-methanol-acetic acid 5:4:1:0.04) of the remaining crude product followed by crystallization from toluene gave 450 mg (60%) of 2-(3,5-dinitrobenzyl)acrylic acid as yellow needles. ¹H NMR (CDCl₃, 300 MHz) δ 8.93, (t, 1H, J = 2.1 Hz), 8.43, (d, 2H, J = 2.1 Hz), 6.56, (s, 1H), 5.93, (m, 1H), 3.85, (s, 2H).

Many of the allylic aromatic compounds produced by the process of the invention, in particular the products of Examples 1-4, 8-14, 16, 18 and 23-27 above, are new compounds and are a further feature of the invention.

One group of these new compounds can be represented by general formula (3): 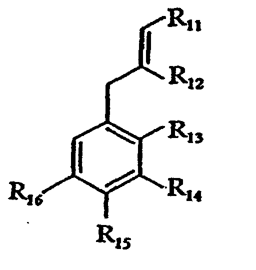 in which:
R₁₁ is H or -COOH,
R₁₂ is H or Br or a phenyl, methyl or -CH₂OCOCH₃ group,
R₁₃ is H, Br or Cl or a methyl, -CF₃, CN or benzoyl group
R₁₄ and R₁₆ are, independently, H or Br or a NO₂, -CF₃, -COOC₂H₅ or -COCH₃ group, and
R₁₅ is H or a NO₂ group,
and two of R₁₃-R₁₆ are H and two are other than H, excluding the compound where R₁₁, R₁₂, R₁₃ and R₁₅ are all H and R₁₄ and R₁₆ are both -CF₃ (this last compound being disclosed by Porwisiak at al. in Chemische Berichte, Vol 129, 1996, pp. 233-235).

R₁₁ in these compounds is usually H and R₁₂ is preferably H, or Br or phenyl. R₁₃ is preferably H, Br, Cl, methyl or -CF₃, R₁₅ is preferably H or NO₂ and R₁₄ and R₁₆ are H, Br, NO₂, -CF₃ or -COOC₂H₅.

Examples of groups of these compounds are those in which R₁₃ and R₁₅ are H and R₁₄ and R₁₆ are other than H, and those in which R₁₄ and R₁₆ are H and R₁₃ and R₁₅ are other than H. Preferred compounds of these types are those in which at least one, and preferably both, of R₁₄ and R₁₆ is NO₂ (R₁₃ and R₁₅ being H), and those in which at least one of R₁₃ and R₁₅ is NO₂ (R₁₄ and R₁₆ being H).

As indicated above, these compounds are useful as intermediates in the preparation of corresponding 2,3-dihydroxypropyl compounds.

## Claims

1. A process for the preparation of an allylic aromatic compound having at least one electron withdrawing substituent on the aromatic ring in which a correspondingly ring-substituted aromatic amine is reacted first with a nitrite and then with an allylic olefin having an eliminatable terminal substituent.

2. A process according to claim 1 in which the nitrite is t-butylnitrite.

3. A process according to claim 1 or claim 2 in which the ring of the aromatic amine is a phenyl or heterocyclic aromatic group substituted by one or two electron withdrawing groups.

4. A process according to claim 1 or claim 2 in which the aromatic amine is aniline substituted by one or two -NO₂, -COOH, -COOCH₃, -COOC₂H₅, -CONH₂, -CONHPh, -COCH₃, -CN or -CF₃ groups.

5. A process according to any of the preceding claims in which the allylic olefin has the formula R^{a}L where R^{a} is an optionally substituted 2,3-alkenyl group and L is an eliminatable terminal substituent.

6. A process according to any one of claims 1 to 4 in which the allylic olefin is allyl bromide.

7. Compounds of formula (3): 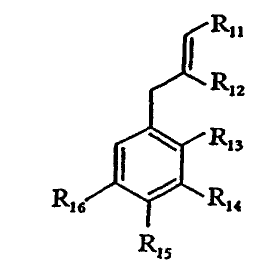 in which:
R₁₁ is H or -COOH,
R₁₂ is H or Br or a phenyl, methyl or -CH₂OCOCH₃ group,
R₁₃ is H, Br or Cl or a methyl, -CF₃, CN or benzoyl group,
R₁₄ and R₁₆ are, independently, H or Br or a NO₂, -CF₃, -COOC₂H₅ or -COCH₃ group, and
R₁₅ is H or a NO₂ group,
and two of R₁₃-R₁₆ are H and two are other than H, excluding the compound where R₁₁, R₁₂, R₁₃ and R₁₅ are all H and R₁₄ and R₁₆ are both -CF₃.

8. Compounds according to claim 7 in which R₁₃ and R₁₅ are H and R₁₄ and R₁₆ are other than H, or in which R₁₄ and R₁₆ are H and R₁₃ and R₁₅ are other than H.

9. Compounds according to claim 7 in which at least one, and preferably both, of R₁₄ and R₁₆ is NO₂ (R₁₃ and R₁₅ being H), or in which at least one of R₁₃ and R₁₅ is NO₂ (R₁₄ and R₁₆ being H).

10. A compound according to claim 7, which is 1-allyl-3, 5-dinitrobenzene.

## Patentansprüche

1. Verfahren zur Herstellung einer allylischen, aromatischen Verbindung mit mindestens einem Elektronen-ziehenden Substituenten am Aromatenring, in welchem ein dementsprechend ringsubstituiertes aromatisches Amin zuerst mit einem Nitrit, und anschließend mit einem allylischen Olefin, das einen abspaltbaren terminalen Substituenten besitzt, umgesetzt wird.

2. Verfahren nach Anspruch 1, in welchem das Nitrit t-Butylnitrit ist.

3. Verfahren nach Anspruch 1 oder 2, in welchem der Ring des aromatischen Amins eine Phenyl- oder eine heterocyclische aromatische Gruppe ist, welche mit einer oder zwei Elektronen-ziehenden Gruppen substituiert ist.

4. Verfahren nach Anspruch 1 oder Anspruch 2, in welchem das aromatische Amin ein Anilin ist, das mit einer oder zwei -NO₂, -COOH, -COOCH₃, -COOC₂H₅, -CONH₂, -CONHPh, -COCH₃, -CN oder -CF₃-Gruppen substituiert ist.

5. Verfahren nach irgendeinem der vorangehenden Ansprüche, in welchem das allylische Olefin die Formel R^{a}L besitzt, worin R^{a} eine wahlweise substituierte 2,3-Alkenylgruppe ist und L ein abspaltbarer terminaler Substituent ist.

6. Verfahren nach irgendeinem der Ansprüche 1 bis 4, in welchem das allylische Olefin Allylbromid ist.

7. Verbindungen der Formel (3): 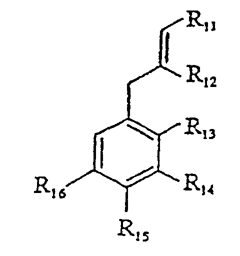 in welcher:
R₁₁ H oder -COOH ist,
R₁₂ H oder Br oder eine Phenyl-, Methyl- oder -CH₂OCOCH₃-Gruppe ist,
R₁₃ H, Br oder Cl oder eine Methyl-, -CF₃-, CN- oder Benzoyl-Gruppe ist.
R₁₄ und R₁₆ unabhängig voneinander H oder Br oder eine NO₂-, -CF₃-, -COOC₂H₅oder -COCH₃-Gruppe sind, und
R₁₅ H oder eine NO₂-Gruppe ist,
und zwei von R₁₃-R₁₆ H sind und zwei etwas anderes als H sind, ausgenommen die Verbindung, in der R₁₁, R₁₂, R₁₃ und R₁₅ alle H und R₁₄ und R₁₆ beide -CF₃ sind.

8. Verbindungen nach Anspruch 7, in denen R₁₃ und R₁₅ H sind und R₁₄ und R₁₆ etwas anderes als H sind, oder in denen R₁₄ und R₁₆ H sind und R₁₃ und R₁₅ etwas anderes als H sind.

9. Verbindungen nach Anspruch 7, in denen mindestens eines, vorzugsweise beide R₁₄ und R₁₆ NO₂ sind (R₁₃ und R₁₅ sind H), oder in denen mindestens eines von R₁₃ und R₁₅ NO₂ ist (R₁₄ und R₁₆ sind H).

10. Verbindung nach Anspruch 7, nämlich 1-Allyl-3,5-dinitrobenzol.

## Revendications

1. Procédé pour la préparation d'un composé aromatique allylique ayant au moins un substituant attracteur d'électrons sur le cycle aromatique, dans lequel on fait réagir une amine aromatique à cycle substitué de façon correspondante d'abord avec un nitrite puis avec une oléfine allylique ayant un substituant terminal éliminable.

2. Procédé selon la revendication 1, dans lequel le nitrite est le nitrite de t-butyle.

3. Procédé selon la revendication 1 ou la revendication 2, dans lequel le cycle de l'amine aromatique est un groupe aromatique phénylique ou hétérocyclique substitué par un ou deux groupes attracteurs d'électrons.

4. Procédé selon la revendication 1 ou la revendication 2, dans lequel l'amine aromatique est l'aniline substituée par un ou deux groupes -NO₂, -COOH, -COOCH₃, -COOC₂H₅, -CONH₂, -CONHPh, -COCH₃, -CN ou -CF₃.

5. Procédé selon l'une quelconque des revendications précédentes, dans lequel l'oléfine allylique a la formule R^{a}L où R^{a} est un groupe 2,3-alcényle facultativement substitué et L est un substituant terminal éliminable.

6. Procédé selon l'une quelconque des revendications 1 à 4, dans lequel l'oléfine allylique est le bromure d'allyle.

7. Composés de formule (3) : 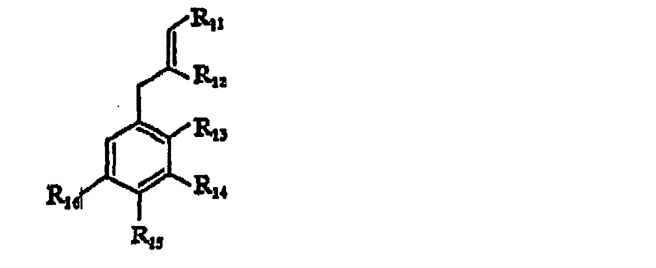 dans laquelle :
R₁₁ est H ou -COOH,
R₁₂ est H ou Br ou un groupe phényle, méthyle ou -CH₂OCOCH₃,
R₁₃ est H, Br ou Cl ou un groupe méthyle, -CF₃, CN ou benzoyle,
R₁₄ et R₁₆ sont, indépendamment, H ou Br ou un groupe NO₂, -CF₃, -COOC₂H₅ ou -COCH₃, et
R₁₅ est H ou un groupe NO₂,
et deux parmi R₁₃ à R₁₆ sont H et deux sont autres que H, à l'exclusion du composé dans lequel R₁₁, R₁₂, R₁₃ et R₁₅ sont tous H et R₁₄ et R₁₆ sont tous deux -CF₃.

8. Composés selon la revendication 7, dans lesquels R₁₃ et R₁₅ sont H et R₁₄ et R₁₆ sont autres que H, ou dans lesquels R₁₄ et R₁₆ sont H et R₁₃ et R₁₅ sont autres que H.

9. Composés selon la revendication 7, dans lesquels au moins un, et de préférence les deux, parmi R₁₄ et R₁₆ est NO₂ (R₁₃ et R₁₅ étant H), ou dans lesquels au moins l'un parmi R₁₃ et R₁₅ est NO₂ (R₁₄ et R₁₆ étant H).

10. Composé selon la revendication 7, qui est le 1-allyl-3,5-dinitrobenzène.
